Europäisches Patentamt

European Patent Office

Office européen des brevets

(11) Veröffentlichungsnummer : **0 050 769
B1**

# (12) EUROPÄISCHE PATENTSCHRIFT

(45) Veröffentlichungstag der Patentschrift :
13.06.84

(21) Anmeldenummer : 81108115.7

(22) Anmeldetag : 09.10.81

(51) Int. Cl.³ : **A 61 F 5/01**

(54) Stabilisierungsbandage für das Kniegelenk.

(30) Priorität : 29.10.80 DE 3040595

(43) Veröffentlichungstag der Anmeldung :
05.05.82 Patentblatt 82/18

(45) Bekanntmachung des Hinweises auf die Patenterteilung : 13.06.84 Patentblatt 84/24

(84) Benannte Vertragsstaaten :
AT BE CH FR GB IT LI LU NL SE

(56) Entgegenhaltungen :
DE-A- 2 133 440

(73) Patentinhaber : von Torklus, Detlef, Prof. Dr.
Jungfrauenthal 20
D-2000 Hamburg 13 (DE)

Thum, Oskar
Bebelallee 116
D-2000 Hamburg 60 (DE)

Wilharm, Friedrich
Martinistrasse 52
D-2000 Hamburg 20 (DE)

Lodenkämper, Harald
Drosselstieg 12
D-2359 Henstedt-Ulzburg (DE)

(72) Erfinder : von Torklus, Detlef, Prof. Dr.
Jungfrauenthal 20
D-2000 Hamburg 13 (DE)
Erfinder : Thum, Oskar
Bebelallee 116
D-2000 Hamburg 60 (DE)
Erfinder : Wilharm, Friedrich
Martinistrasse 52
D-2000 Hamburg 20 (DE)
Erfinder : Lodenkämper, Harald
Drosselstieg 12
D-2359 Henstedt-Ulzburg (DE)

(74) Vertreter : Minetti, Ralf, Dipl.-Ing.
Ballindamm 15
D-2000 Hamburg 1 (DE)

EP 0 050 769 B1

**Beschreibung**

Die Erfindung betrifft eine Stabilisierungsbandage für das Kniegelenk, mit einer seitlich am Kniegelenk anliegenden Stützscheibe, mit einer Schiene, die aus zwei auf der anderen Seite des Beines am Oberschenkel und am Unterschenkel anliegenden Stützplatten und aus zwei die Stützplatten tragenden, drehgelenkig miteinander verbundenen Tragarmen besteht, mit einer U-förmigen, bügelförmigen, an einem Ende die Stützscheibe für das Kniegelenk drehbar tragenden Verbindung zwischen der Stützscheibe und der Schiene und mit zwei an den Stützplatten der Tragarme angeordneten elastischen Bandagebreitbändern nach DE-A-21 33 440.

Kniebandagen werden benutzt zur Stützung des Kniegelenkes bei Verschleißerscheinungen oder Verletzungen des Kniegelenkes wie Bänderlockerungen oder Bänderrissen und auch als nachoperative Bandage. Die Bandage ist dazu so beschaffen, daß der Träger nach ihrer Anlage noch das Kniegelenk bewegen kann, jedoch durch eine seitlich angeordnete Stützscheibe eine zusätzliche Führung hat, die ein seitliches Ausknicken verhindert. Diese Stützscheibe kann je nach Bedarf auf der Innen- oder Außenseite des Knies wirken. Die zusätzliche Anordnung von Stützplatten wurde für notwendig erachtet, um durch eine Art von Dreipunktabstützung die geeignete Lage der Stützscheibe für das Knie auch bei vielfachen Bewegungen des Beines sicherzustellen.

Bei der bekannten Bandage sind die Stützplatten der gelenkig miteinander verbundenen Tragarme, die endseitig die beiden Stützplatten tragen, durch zwei jeweils gewunden geformte und rund um die beiden Körperglieder bis auf die entgegengesetzte Seite verlaufende Bügel mit der Stützscheibe verbunden, von denen beim Tragen der Bandage bei einer bevorzugten Ausführungsform der eine Bügel auf dem Schienenbein des Unterschenkels aufliegt und der andere Bügel auf der Oberseite des Oberschenkels aufliegt. Das setzt eine sehr genaue Ausformung der in sich steifen Bügel in individueller Anpassung an die Form und Größe des Oberschenkels und Unterschenkels voraus. Eine derartige Ausformung ist jedoch nur möglich, durch eine Fertigung nach einem vom Bein abgenommenen Gipsabdruck. Hinzu kommt, daß Bügel beim Gehen hinderlich sind, wenn sie auf der Vorderseite des Beines aufliegen, und zwar insbesondere der untere Bügel, der beim Gehen auf das Schienenbein einen Druck ausübt. Ein weiterer Nachteil ergibt sich in ästhetischer Hinsicht insoweit, als sich die Bandage beim Tragen einer enganliegenden Hose für Dritte deutlich sichtbar abzeichnet, sofern die Bügel auf der Vorderseite der Körperteile getragen werden. Schließlich wurde es als Nachteil der bekannten Bandage empfunden, daß diese verhältnismäßig schwer ist, aufgrund der Vielzahl der einzelnen individuell angepaßten Träger und Verbindungsstützen, die noch von einem Gummischlauch umgeben sind, damit sie beim Tragen nicht so störend wirken.

Ein wesentlicher Nachteil ist jedoch darin zu sehen, daß die bekannte Stabilisierungsbandage durch die zwei in sich steifen Bügel eine sehr steife Konstruktion bildet, für deren Herstellung es einer sehr genauen Anpassung bedarf, wenn durch die Stützscheibe in verschiedenen Stellungen des Knies seitlich ein Druck ausgeübt werden soll, der sich nicht ausschließlich aus der Elastizität der Polsterung der Scheibe ergibt. Dafür ist es zweckmäßig, wenn die Stützscheibe seitlich federnd am Kniegelenk anliegt.

Aufgabe der Erfindung ist es, eine Stabilisierungsbandage für das Kniegelenk zu schaffen, die weniger aufwendig ist in der Anpassung an den Träger und diesen weniger stark behindert. Nach der Erfindung ist dafür vorgesehen, daß bei einer Stabilisierungsbandage der eingangs genannten Art die Verbindung zwischen der Stützscheibe und der Schiene aus nur einem U-förmigen Bügel besteht, der am unteren Tragarm befestigt ist und an der Wade des Unterschenkels anliegt. Eine derartige Bandage ist leichter in der Konstruktion und behindert weniger beim Tragen. Außerdem wirkt sie in ästhetischer Hinsicht nicht nachteilig und ist billiger herzustellen, da es nicht einer besonderen individuellen Anpassung in der Gestaltung ihrer Einzelteile bedarf, sondern stattdessen einige wenige verschiedene Größen ausreichen, um alle Bedürfnisse befriedigen zu können.

Weitere Vorteile ergeben sich aus zusätzlichen Merkmalen, die Gegenstand der Ansprüche 2 bis 5 bilden.

Ein Ausführungsbeispiel der Erfindung ist nachstehend unter Bezugnahme auf eine Zeichnung erläutert. In der Zeichnung zeigen:

Figur 1: Eine Stabilisierungsbandage in perspektivischer Darstellung,

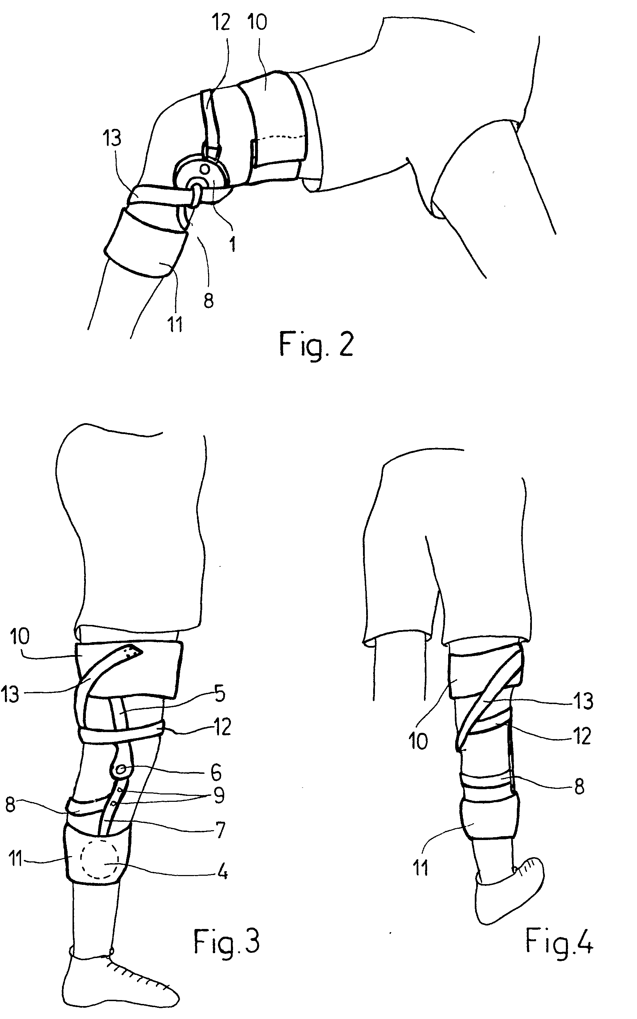

Figur 2: Die Innenseite einer Bandage mit Stützscheibe,

Figur 3: Die Außenseite einer Bandage mit den Trägern und Drehgelenk und

Figur 4: Die Rückansicht der Bandage.

Die in der Zeichnung wiedergegebene Stabilisierungsbandage besitzt eine Stützscheibe 1 für das Kniegelenk, die mit einer Auspolsterung 2 versehen ist. Für den Halt der Stützscheibe 1 am Knie sind zwei weitere Abstützungen auf der gegenüberliegenden Seite des Beines vorgesehen, zu denen zwei Stützplatten 3 und 4 gehören. Die Stützplatte 3 ist endseitig auf einem Träger 5 befestigt, der durch ein Gelenk 6 mit einem zweiten Träger 7 verbunden ist, welcher seinerseits die zweite Stützplatte 4 trägt. Für die Verbindung mit der Stützscheibe 1 ist ein im wesentlichen U-förmiger Bügel 8 vorgesehen, der durch Nieten 9 mit dem untenstehenden Träger 7 fest verbunden ist und die Stützscheibe 1 drehbar trägt. Die Träger 5 und 7 und der U-förmige Bügel 8 bestehen aus einem dünnen Stahlblech, das vorzugsweise verchromt ist. Zur weiteren Stabilisierung sind die Teile 5, 7 und 8 vorzugsweise gewölbt bzw. im Querschnitt gerundet ausgebildet.

Für die Halterung der Bandage am Bein eines Menschen ist ein erstes oberes Bandagebreitband 10 vorgesehen, das zu öffnen ist und einen Klettenbandverschluß 16 aufweist, wie auch das zweite untenliegende Bandagebreitband 11. Zusätzlich ist ein in seiner Länge einstellbarer Riemen 12 aus elastischem Material vorgesehen, der den Oberschenkel umschließt. Ein zweiter Riemen 13 verläuft schraubenlinienförmig von der unteren Breitbandbandage 11 durch eine Schlaufe 15 an der Stützscheibe 1 über diese hinweg zur oberen Stützplatte 3 bzw. der oberen Breitbandbandage 10, so daß er sich über das Schienbein des Trägers hinweg und oberhalb der Kniekehle unter dem Oberschenkel hindurch erstreckt. Auch dieses Gurtband 13 besteht aus einem elastischen Material und ist mit einem Klettenbandverschluß versehen, um den Druck bzw. die Zugkraft den jeweiligen Bedürfnissen entsprechend einstellen zu können.

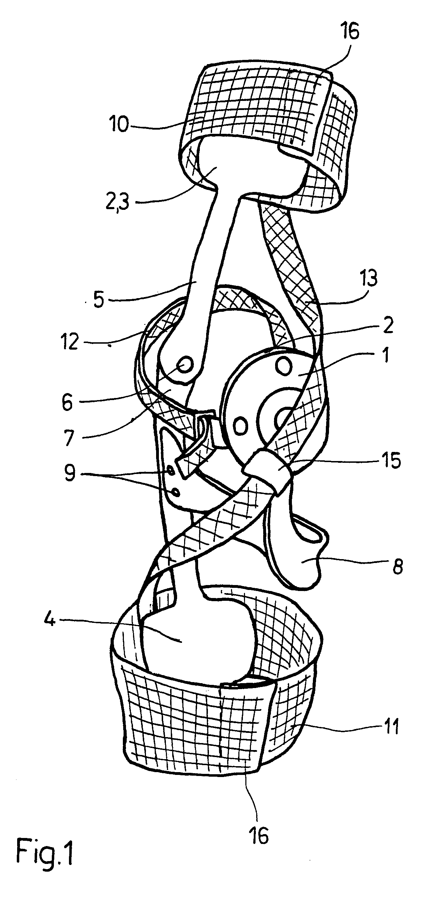

Die beiden Träger 5 und 7 sind nur bis höchstens 90° zueinander schwenkbar und zwar bei der in Figur 1 wiedergegebenen Lage im Sinne einer Schwenkung des oberen Trägers 5 im Uhrzeigersinn.

**Ansprüche**

1. Stabilisierungsbandage für das Kniegelenk
   — mit einer seitlich am Kniegelenk anliegenden Stützscheibe (1),
   — mit einer Schiene, die aus zwei auf der anderen Seite des Beines am Oberschenkel und am Unterschenkel anliegenden Stützplatten (3, 4) und aus zwei die Stützplatten (3, 4) tragenden, drehgelenkig (6) miteinander verbundenen Tragarmen (5, 7) besteht,
   — mit einer U-förmigen, bügelförmigen, an einem Ende die Stützscheibe (1) für das Kniegelenk drehbar tragenden Verbindung zwischen der Stützscheibe (1) und der Schiene (3 bis 7)
   — und mit zwei an den Stützplatten (3, 4) der Tragarme (5, 7) angeordneten elastischen Bandagebreitbändern (10, 11),
   dadurch gekennzeichnet daß die Verbindung zwischen der Stützscheibe (1) und der Schiene (3 bis 7) aus nur einem U-förmigen Bügel (8) besteht, der am unteren Tragarm (7) befestigt ist und an der Wade des Unterschenkels anliegt.

2. Stabilisierungsbandage nach Anspruch 1, dadurch gekennzeichnet, daß die Stützscheibe (1) des Kniegelenkes einen elastischen, unterteilten Riemen (12) zum Umschlingen des Oberschenkels trägt und eine Führung (15) für einen elastischen Gurt (13), der sich schraubenlinienförmig von der unteren Stützplatte (4) über das Schienbein des Trägers hinweg außen über die Stützscheibe (1) des Kniegelenkes und um die Rückseite des Oberschenkels herum zum oberen Bandagebreitband (10) erstreckt.

3. Stabilisierungsbandage nach Anspruch 2, dadurch gekennzeichnet, daß alle Riemengurte und Bandagebänder (10, 11, 12, 13) mit Klettenbandverschlüssen (16) versehen sind.

4. Stabilisierungsbandage nach einem oder mehreren der vorhergehenden Ansprüche, dadurch gekennzeichnet, daß die Tragarme (5, 7) und der Bügel (8) aus einem profilierten, verchromten Stahlblech bestehen.

5. Stabilisierungsbandage nach einem oder mehreren der vorhergehenden Ansprüche, dadurch gekennzeichnet, daß die beiden Tragarme (5, 7) nur bis höchstens 90° zueinander schwenkbar sind.

**Claims**

1. Knee brace
   — with a lateral to the knee joint close sitting supporting ring (1),
   — with a bar consisting of two support plates (3, 4) sitting close on the other side of the leg on the thigh and on the leg and of two bearers (5, 7) bearing the support plates (3, 4) connected to each other with a hinge (6),
   — with a U-shaped, stirrup-shaped junction between the supporting ring (1) and the bar (3 to 7) bearing at one end the supporting ring (1) of the knee joint on a rotatable way,
   — with two elastic bandage broad strips (10, 11) placed at the support plates (3, 4) of the bearers (5, 7),
   characterised in that the junction between the supporting ring (1) and the bar (3 to 7) consists of only one U-shaped stirrup (8) attached to the inferior bearer (7) and sitting close to the calf of the leg.

2. Knee brace according to claim 1, characterised in that the supporting ring (1) of the knee joint bears a divided elastic strip (12) to be wound round the thigh and a guide (15) for an elastic belt (13) which extends helix-shaped from the inferior support plate (4) to the superior bandage broad strip (10) via the bearer's tibia on the exterior side over the supporting ring (1) of the knee joint and around the back of the thigh.

3. Knee brace according to claim 2, characterised in that all strap belts and bandage strips (10, 11, 12, 13) are provided with Velcro strip fasteners (16).

4. Knee brace according to one or several preceding claims, characterised in that the bearers (5, 7) and the stirrup (8) consist of profiled chromium-plated sheet steel.

5. Knee brace according to one or several preceding claims, characterised in that both bearers (5, 7) are only slewable to each other up to 90° at the most.

**Revendications**

1. Orthèse du genou
   — avec un disque de maintien (1) contigu

3

latéralement à l'articulation du genou,
— avec une tige se composant de deux plaques de maintien (3, 4) se trouvant sur l'autre côté de la jambe et étant contiguës à la cuisse et au bas de la jambe et de deux bras-supports (5, 7) reliés l'un à l'autre par une charnière (6) et portant les plaques de maintien (3, 4),
— avec une jonction en U en étrier entre le disque de maintien (1) et la tige (3 à 7) portant de façon pivotante à une extrémité le disque de maintien (1) pour l'articulation du genou,
— avec deux larges bandes de bandage (10, 11) élastiques placées sur les plaques de maintien (3, 4) des bras-supports (5, 7), caractérisée en ce que la jonction entre le disque de maintien (1) et la tige (3 à 7) ne comprend qu'un étrier (8) en forme de U qui est fixé au bras-support inférieur (7) et qui est contigu au mollet du bas de la jambe.

2. Orthèse du genou selon la revendication 1, caractérisée en ce que le disque de maintien (1) de l'articulation du genou porte une courroie (12) élastique divisée pour entourer la cuisse et un guide (15) pour une sangle élastique (13) qui s'étend en forme d'hélice de la plaque de maintien inférieure (4) jusqu'à la bande de bandage supérieure (10) en passant par le tibia du porteur à l'extérieur par le disque de maintien (1) de l'articulation du genou et autour de la partie dorsale de la cuisse.

3. Orthèse du genou selon la revendication 2, caractérisée en ce que toutes les sangles des courroies et les bandes de bandage (10, 11, 12, 13) sont pourvues de fermetures en bande Velcro (16).

4. Orthèse du genou selon une ou plusieurs revendications précédentes, caractérisée en ce que les bras-supports (5, 7) et l'étrier (8) sont en tôle d'acier chromée profilée.

5. Orthèse du genou selon une ou plusieurs revendications précédentes, caractérisée en ce que les deux bras-supports (5, 7) ne peuvent être pivotants l'un par rapport à l'autre que jusque 90° maximum.

Fig.1

Fig. 2

Fig.3

Fig.4